(19) **Europäisches Patentamt**
**European Patent Office**
**Office européen des brevets**

(11) **EP 4 378 949 A1**

(12) **EUROPEAN PATENT APPLICATION**
published in accordance with Art. 153(4) EPC

(43) Date of publication:
**05.06.2024 Bulletin 2024/23**

(21) Application number: **22848687.4**

(22) Date of filing: **29.07.2022**

(51) International Patent Classification (IPC):
*C07K 7/06* (2006.01)    *C07K 7/08* (2006.01)
*A61K 38/08* (2019.01)    *A61K 38/10* (2006.01)
*A61P 25/00* (2006.01)    *A61P 9/12* (2006.01)
*A61P 25/28* (2006.01)    *A61P 25/16* (2006.01)

(86) International application number:
**PCT/CN2022/109048**

(87) International publication number:
**WO 2023/006083 (02.02.2023 Gazette 2023/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.07.2021   CN 202110873573**

(71) Applicant: **Innerse (Zhuhai) Pharmaceutical Co.,
Ltd.**
**Zhuhai City, Guangdong 519031 (CN)**

(72) Inventor: **LI, Jianxiong**
**Wuhan, Hubei 430075 (CN)**

(74) Representative: **Cabinet Laurent & Charras
Le Contemporain
50 Chemin de la Bruyère
69574 Dardilly Cedex (FR)**

(54) **NEUROPROTECTIVE POLYPEPTIDE COMPOUND AND APPLICATION THEREOF**

(57) The present invention relates to the technical field of medicines and disclosed are a neuroprotective polypeptide compound and an application thereof. The neuroprotective polypeptide compound comprises a polypeptide having the following chemical formula and a salt thereof: (M)m-(Lys-Leu-Ser-Ser-Ile-Glu/Asp-Ser/Thr-Asp/Glu-Val/Leu)-(N)n; m and n are integers from 0 to 3, but m and n are not zero at the same time; M is beta-Ala-His, beta-Ala-1-Methyl-His or beta-Ala-3-Methyl-His, and N is beta-Ala-His, beta-Ala-1-Methyl-His or beta-Ala-3-Methyl-His. An intravenous dosage of only 3 mg/kg can unexpectedly achieve a significant therapeutic effect and achieve the same effect as NA1. The polypeptide compound can be used in combination with a hemolytic drug to provide a new possibility for the treatment of stroke.

EP 4 378 949 A1

## Description

### Technical field

The disclosure belongs to the technical field of medicine, and in particular it relates to a neuroprotective polypeptide and application thereof.

### Background

Nerinetide (NA-1) is a neuroprotectant that interferes with postsynaptic density protein 95 (PSD-95) by terminating the production of intracellular NO free radicals. It reduces the infarct area of cerebral ischemia-reperfusion and improves functional outcomes in preclinical ischemic stroke models. For adult patients with acute ischemic stroke due to large vessel occlusion within a 12-hour treatment window, nerinetide at a single dose of 2.6 mg/kg and a maximum dose of 270 mg or saline placebo were randomly given. The primary outcome of the study was a favourable functional outcome 90 days after randomization, defined as a modified Rankin Scale (mRS) score of 0-2; the secondary outcome was neurological disability, functional independence in daily activities, good functional outcome (mRS 0-1) and mortality. A total of 1,105 patients were recruited in the trial, including 549 in the Nerinetide group and 556 in the placebo group. The proportion of patients with an mRS score of 0-2 within 90 days was: 337 (61.4%) in the nerinetide group and 329 (59.2%) in the placebo group. The secondary outcomes were similar between the two groups. The study also found that nerinetide treatment resulted in inhibition of treatment effect in patients receiving alteplase. Serious adverse events occurred equally between groups. See Michael D Hill et al. Efficacy and safety of nerinetide for the treatment of acute ischaemic stroke (ESCAPE-NA1): a multicentre, double-blind, randomized controlled trial. Lancet. 2020, 395(10227), P878-887.

Nerinetide, also called Tat-NR2B9c, is a fusion peptide composed of the C-terminal 9 residues of NMDAR GluN2B subunit and the transmembrane peptide TAT derived from nuclear transport activator protein. It can bind to the PDZ-1 or PDZ-2 domain of PSD-95, thereby inhibiting the NO production by nNOS. Its specific amino acid sequence is: Tyr-Gly-Arg-Lys-Lys-Arg-Arg-Gln-Arg-Arg-Arg-Lys-Leu-Ser-Ser-Ile-Glu-Ser-Asp-Val. The latter part of the sequence, Lys-Leu-Ser-Ser-Ile-Glu-Ser-Asp-Val, is the sequence of NR2B9c and specifically inhibits the NO production by nNOS. The front part of the sequence, Tyr-Gly-Arg-Lys-Lys-Arg-Arg-Gln-Arg-Arg-Arg, improves the bioavailability of NR2B9c. The amino acid sequence of active peptides like NR2B9c consists of 3-25 amino acids from the C terminus of an NMDA receptor or PDZ domain 1 and/or 2 of the PSD-95 receptor linked to an internalization peptide (A. Tasker, T. Doucette, M. Tymianski, K. Mendoza, M. P. Belmares, D. Garman, and P. S. Lu, 2013, United States Patent 8,536,129). This type of active peptides have an amino acid sequence containing [E/D/N/Q]-[S/T]-[D/E/Q/N]-[V/L], such as KLSSIETDV and KLSSIESDV.

Carnosine (L-Carnosine) is a dipeptide composed of two amino acids, β-alanine and L-histidine. Carnosine has a strong anti-oxidative capacity and is beneficial to human body. Carnosine has been shown to clear off reactive oxygen species (ROS) and α-β unsaturated aldehydes formed during oxidative stress by the excessive oxidation of fatty acids on cell membrane. Carnosine has anti-inflammatory, anti-glycation, anti- oxidative and chelating functions, and it can serve as an over-the-counter food supplement with great potentials in the prevention and co-treatment of chronic diseases such as cardiovascular diseases and neurodegenerative diseases. The neuroprotective mechanism of carnosine can prevent permanent cerebral ischemia according to animal experiments. Carnosine is also an important intracellular antioxidant. Carnosine is non-toxic and has a strong anti-oxidative effect, and thus a widespread attention has been paid to it as a new food additive and a medical agent. Carnosine involves in intracelular peroxidative reaction. In addition to the cell membrane peroxidation processes, Carnosine can also inhibit associated intracellular peroxidation reaction. In 1900, Gulewitsch from Russia first discovered carnosine, and then scientists from multiple countries isolated other histidine dipeptide derivatives from different muscular tissues, such as anserine, which is a dipeptide composed of β-alanine and 1-methyl-L-histidine, as well as balenine (also known as Ophidine), which is a dipeptide composed of β-alanine and 3-methyl-L-histidine. The amounts and ratios of these histidine dipeptides are also different among different species, with certain specificity. In addition to muscular tissues, these histidine dipeptides are also found in other types of tissues, such as brain. These carnosine derivatives are water-soluble and have strong and significant functions in anti-oxidation, anti-aging, and lowering uric acid. They have been used as natural antioxidants and uric acid-lowering diet therapy in food industry, and they also have certain neuroprotective effect.

Although carnosine and its derivatives have certain brain-protecting effect, it often requires high dose. Carnosine, anserine and ophidine alone has not achieved great effect. New neuroprotective methods are still urgently needed in this field.

**EP 4 378 949 A1**

**Summary**

The inventors have combined carnosine and carnosine derivatives with active peptides such as NR2B9c, to create novel polypeptides. Surprisingly, these novel polypeptides, which are completely different from Nerinetide, have showed good neuroprotection which is not influenced by thrombolytic agents like alteplase, thus providing new ways for neuroprotective strategies.

Herein provided is a neuroprotective polypeptide compound comprising carnosine, anserine, ophidine, etc. composed of beta-alanine and histidine, 1-methylhistidine, 3-methylhistidine, etc. as well as an active peptide such as NR2B9c. The amino acid sequence of the active peptide is Lys-Leu-Ser-Ser-Ile-Glu/Asp-Ser/Thr-Asp/Glu-Val/Leu, in particular NR2B9c set forth by Lys-Leu-Ser-Ser-Ile-Glu-Ser-Asp-Val. Hence a series of combined polypeptide compounds having the characteristics of carnosine, anserine or ophidine and that of Lys-Leu-Ser-Ser-Ile-Glu/Asp-Ser/Thr-Asp/Glu-Val/Leu. Such peptides unexpectedly penetrate the blood-brain barrier and exhibit good biological activity when administered via intravenous route, thus showing broad prospects in the treatment of neurological diseases, especially brain injury and stroke.

In one respect, herein provided is a neuroprotective polypeptide compound comprising a polypeptide having the following chemical formula and a salt thereof:

(M)m-(Lys-Leu-Ser-Ser-Ile-Glu/Asp-Ser/Thr-Asp/Glu-Val/Leu)-(N)n,

wherein, m is an integer from 0 to 3, n is an integer from 0 to 3, and m and n are not simultaneously zero; M is beta-Ala-His, beta-Ala-1-Methyl-His or beta-Ala-3-Methyl-His, N is beta-Ala-His, beta-Ala-1-Methyl-His or beta-Ala-3-Methyl-His. In the formula, Glu/Asp means that the amino acid at the indicated position may be either Glu or Asp, Ser/Thr means that the amino acid at the indicated position may be either Ser or Thr, Asp/Glu means that the amino acid at the indicated position may be either Asp or Glu, and Val/Leu means that the amino acid at the indicated position may be either Val or Leu.

A preferable example of Lys-Leu-Ser-Ser-Ile-Glu/Asp-Ser/Thr-Asp/Glu-Val/Leu includes Lys-Leu-Ser-Ser-Ile-Glu-Ser-Asp-Val.

In some embodiments, the neuroprotective polypeptide compound of the disclosure comprises a polypeptide having the following chemical formula and a salt thereof:

beta-Ala-His-Lys-Leu-Ser-Ser-Ile-Glu/Asp-Ser/Thr-Asp/Glu-Val/Leu;
Lys-Leu-Ser-Ser-Ile-Glu/Asp-Ser/Thr-Asp/Glu-Val/Leu-beta-Ala-His;
beta-Ala-His-beta-Ala-His-Lys-Leu-Ser-Ser-Ile-Glu/Asp-Ser/Thr-Asp-Val/Leu;

Lys-Leu-Ser-Ser-Ile-Glu/Asp-Ser/Thr-Asp/Glu-Val/Leu-beta-Ala-His-beta-Ala-His;

beta-Ala-His-Lys-Leu-Ser-Ser-Ile-Glu/Asp-Ser/Thr-Asp/Glu-Val/Leu-beta-Ala-His;

beta-Ala-His-beta-Ala-His-beta-Ala-His-Lys-Leu-Ser-Ser-Ile-Glu/Asp-Ser/Thr-Asp-Val/Leu;

beta-Ala-His-beta-Ala-His-Lys-Leu-Ser-Ser-Ile-Glu/Asp-Ser/Thr-Asp-Val/Leu-beta-Ala-His;

beta-Ala-His-Lys-Leu-Ser-Ser-Ile-Glu/Asp-Ser/Thr-Asp/Glu-Val/Leu-beta-Ala-His-beta-Ala-His; and

Lys-Leu-Ser-Ser-Ile-Glu/Asp-Ser/Thr-Asp/Glu-Val/Leu-beta-Ala-His-beta-Ala-His-beta-Ala-His;

wherein, His is His, 1-Methyl-His or 3-Methyl-His, and hence the preceding M or N corresponds to carnosine, anserine and ophidine, respectively.

In particular, the neuroprotective polypeptide compound of the disclosure comprises a polypeptide having the following chemical formula and a salt thereof:

beta-Ala-His-Lys-Leu-Ser-Ser-Ile-Glu-Ser-Asp-Val;
beta-Ala-His-Lys-Leu-Ser-Ser-Ile-Asp-Ser-Asp-Val;
beta-Ala-His-Lys-Leu-Ser-Ser-Ile-Glu-Thr-Asp-Val;
beta-Ala-His-Lys-Leu-Ser-Ser-Ile-Glu-Thr-Glu-Val;
beta-Ala-His-Lys-Leu-Ser-Ser-Ile-Glu-Ser-Glu-Val;
beta-Ala-His-Lys-Leu-Ser-Ser-Ile-Asp-Thr-Asp-Val;
beta-Ala-His-Lys-Leu-Ser-Ser-Ile-Asp-Ser-Glu-Val;
beta-Ala-His-Lys-Leu-Ser-Ser-Ile-Asp-Thr-Glu-Val;
Lys-Leu-Ser-Ser-Ile-Glu-Ser-Asp-Val-beta-Ala-His;
Lys-Leu-Ser-Ser-Ile-Asp-Ser-Asp-Val-beta-Ala-His;
Lys-Leu-Ser-Ser-Ile-Glu-Thr-Asp-Val-beta-Ala-His;
Lys-Leu-Ser-Ser-Ile-Glu-Thr-Glu-Val-beta-Ala-His;
Lys-Leu-Ser-Ser-Ile-Glu-Ser-Glu-Val-beta-Ala-His;
Lys-Leu-Ser-Ser-Ile-Asp-Thr-Asp-Val-beta-Ala-His;
Lys-Leu-Ser-Ser-Ile-Asp-Ser-Glu-Val-beta-Ala-His;
Lys-Leu-Ser-Ser-Ile-Asp-Thr-Glu-Val-beta-Ala-His;
beta-Ala-(1-methyl-His)-Lys-Leu-Ser-Ser-Ile-Glu-Ser-Asp-Val;
beta-Ala-(1-methyl-His)-Lys-Leu-Ser-Ser-Ile-Asp-Ser-Asp-Val;
beta-Ala-(1-Methyl-His)-Lys-Leu-Ser-Ser-Ile-Glu-Thr-Asp-Val;
beta-Ala-(1-Methyl-His)-Lys-Leu-Ser-Ser-Ile-Glu-Thr-Glu-Val;
beta-Ala-(1-Methyl-His)-Lys-Leu-Ser-Ser-Ile-Glu-Ser-Glu-Val;
beta-Ala-(1-Methyl-His)-Lys-Leu-Ser-Ser-Ile-Asp-Thr-Asp-Val;
beta-Ala-(1-Methyl-His)-Lys-Leu-Ser-Ser-Ile-Asp-Ser-Glu-Val;
beta-Ala-(1-Methyl-His)-Lys-Leu-Ser-Ser-Ile-Asp-Thr-Glu-Val;
Lys-Leu-Ser-Ser-Ile-Glu-Ser-Asp-Val-beta-Ala-(1-Methyl-His);
Lys-Leu-Ser-Ser-Ile-Asp-Ser-Asp-Val-beta-Ala-(1-Methyl-His);
Lys-Leu-Ser-Ser-Ile-Glu-Thr-Asp-Val-beta-Ala-(1-Methyl-His);
Lys-Leu-Ser-Ser-Ile-Glu-Thr-Glu-Val-beta-Ala-(1-Methyl-His);
Lys-Leu-Ser-Ser-Ile-Glu-Ser-Glu-Val-beta-Ala-(1-Methyl-His);
Lys-Leu-Ser-Ser-Ile-Asp-Thr-Asp-Val-beta-Ala-(1-Methyl-His);
Lys- Leu-Ser-Ser- Ile-Asp-Ser-Glu- Val-beta-Ala-(1- Methyl-His);
Lys-Leu-Ser-Ser-Ile-Asp-Thr-Glu-Val-beta-Ala-(1-Methyl-His);
beta-Ala-(3-methyl-His)-Lys-Leu-Ser-Ser-Ile-Glu-Ser-Asp-Val;
beta-Ala-(3-methyl-His)-Lys-Leu-Ser-Ser-Ile-Asp-Ser-Asp-Val;
beta-Ala-(3-Methyl-His)-Lys-Leu-Ser-Ser-Ile-Glu-Thr-Asp-Val:

beta-Ala-(3-Methyl-His)-Lys-Leu-Ser-Ser-Ile-Glu-Thr-Glu-Val;
beta-Ala-(3-Methyl-His)-Lys-Leu-Ser-Ser-Ile-Glu-Ser-Glu-Val;
beta-Ala-(3-Methyl-His)-Lys-Leu-Ser-Ser-Ile-Asp-Thr-Asp-Val;
beta-Ala-(3-Methyl-His)-Lys-Leu-Ser-Ser-Ile-Asp-Ser-Glu-Val;
beta-Ala-(3-Methyl-His)-Lys-Leu-Ser-Ser-Ile-Asp-Thr-Glu-Val;
Lys-Leu-Ser-Ser-Ile-Glu-Ser-Asp-Val-beta-Ala-(3-Methyl-His);
Lys-Leu-Ser-Ser-Ile-Asp-Ser-Asp-Val-beta-Ala-(3-Methyl-His);
Lys-Leu-Ser-Ser-Ile-Glu-Thr-Asp-Val-beta-Ala-(3-Methyl-His);
Lys-Leu-Ser-Ser-Ile-Glu-Thr-Glu-Val-beta-Ala-(3-Methyl-His);
Lys-Leu-Ser-Ser-Ile-Glu-Ser-Glu-Val-beta-Ala-(3-Methyl-His);
Lys-Leu-Ser-Ser-Ile-Asp-Thr-Asp-Val-beta-Ala-(3-Methyl-His);
Lys-Leu-Ser-Ser-Ile-Asp-Ser-Glu-Val-beta-Ala-(3-Methyl-His);

Lys-Leu-Ser-Ser-Ile-Asp-Thr-Glu-Val-beta-Ala-(3-Methyl-His).

Preferably, the neuroprotective polypeptide compound of the present disclosure comprises a polypeptide having the following chemical formula and a salt thereof:

beta-Ala-His-Lys-Leu-Ser-Ser-Ile-Glu-Ser-Asp-Val;
Lys-Leu-Ser-Ser-Ile-Glu-Ser-Asp-Val-beta-Ala-His;
beta-Ala-(1-methyl-His)-Lys-Leu-Ser-Ser-Ile-Glu-Ser-Asp-Val;
Lys-Leu-Ser-Ser-Ile-Glu-Ser-Asp-Val-beta-Ala-(1-Methyl-His);
beta-Ala-(3-methyl-His)-Lys-Leu-Ser-Ser-Ile-Glu-Ser-Asp-Val;
Lys-Leu-Ser-Ser-Ile-Glu-Ser-Asp-Val-beta-Ala-(3-Methyl-His).

More preferably, the neuroprotective polypeptide compound of the present disclosure comprises a polypeptide having the following chemical formula and a salt thereof:

beta-Ala-His-Lys-Leu-Ser-Ser-Ile-Glu-Ser-Asp-Val;
Lys-Leu-Ser-Ser-Ile-Glu-Ser-Asp-Val-beta-Ala-His.

Most preferably, the neuroprotective polypeptide compound of the present disclosure comprises a polypeptide having the following chemical formula and s salt thereof:
beta-Ala-His-Lys-Leu-Ser-Ser-Ile-Glu-Ser-Asp-Val.
In another respect, provided herein is use of an aforementioned neuroprotective polypeptide compound in the manufacture of a medicament, especially a medicament for treating a neurological disease. Also provided is an aforementioned neuroprotective polypeptide compound for use in treating a neurological disease. Also provided is a method of treating a neurological disease using an aforementioned neuroprotective polypeptide compound.
In particular, the neurological disease may be ischemic stroke, hemorrhagic stroke, brain trauma, Alzheimer's disease, Parkinson's disease or other neurodegenerative diseases. Preferably, the neurological disease is ischemic stroke.
The neuroprotective polypeptide compound provided by the present disclosure may be formulated as a medicament. Said medicament may be administered via injection, oral, sublingual, spray or anal way, etc., and preferably injection. In particular, the medicament for injection may be powder or liquid. Further, the medicament may be administered intravenously. The medicament may also be used as an active ingredient to be formulated into other dosage forms suitable to the desired medical use. As a neuroprotective agent, the formulation may include an oral formulation and a sublingual formulation. As a first aid drug for stroke, the formulation may include a spray formulation, an anal formulation etc. to facilitate the treatment of patients without action capability.
Further, the medicament comprises a pharmaceutically acceptable diluent or/and carrier.
In another aspect, provided herein is a method for preparing various neuroprotective polypeptide compounds of the present disclosure through solid-phase synthesis, although it would be more convenient to use liquid-phase synthesis or fragment synthesis for some of the polypeptides. Salifying a polypeptide is one of the common means to improve the physical and chemical properties of the polypeptide as well as enhance the druggability. The medicament can be any form of salts.
In another respect, herein provided is a combination of the neuroprotective polypeptide compound herein and a thrombolytic drug.
The thrombolytic drug may be the first-generation thrombolytic drugs such as streptokinase and urokinase, the second-generation thrombolytic drugs such as tissue-type plasminogen activator (tPA) alteplase and prourokinase, or the third-generation thrombolytic drugs based on a recombinant human tissue-type plasminogen activator (rtPA). Many of such commercial products are known in the art, such as urokinase for injection (Tianjin Biochemical Pharmaceutical Co., Ltd.), recombinant streptokinase for injection (such as Sikaitong), alteplase for injection (such as Aitongli), recombinant human TNK tissue-type plasminogen activator for injection (such as Mingfule).
The neuroprotective polypeptide compound and the thrombolytic drug described herein may be administered separately either sequentially or simultaneously, or mixed at any suitable ratio as a combination.
The combination may be used to prepare a medicament, especially for the treatment of a neurological disease.
The neurological disease may be ischemic stroke, hemorrhagic stroke, brain trauma, Alzheimer's disease, Parkinson's disease or other neurodegenerative diseases. Preferably, the neurological disease is ischemic stroke.
In order to determine use of the synthetic peptides provided herein in a neurological disease, SD rats were used to prepare a rat model of cerebral ischemia via middle cerebral artery occlusion (MCAO). These rats were injected intravenously 1-2 hours after ischemia, and subjected for a behavioral observation and scoring after 22-24 hours. After the behavioral observation, the rats were euthanized and their brains were removed, cut to sections and stained with TTC for quantitative analysis, and the cerebral infarct volume % was calculated.

The present inventor inventively combines the active peptides such as NR2B9c with carnosine, anserine or ophidine, etc. to develop a combined polypeptide, which unexpectedly passes the blood-brain barrier through intravenous injection, enters the brain, and plays an effective role in treating a neurological disease relative to any part of the combined polypeptide. Such synthetic polypeptides are preferably used in the treatment of ischemic stroke, hemorrhagic stroke, brain trauma, Alzheimer's disease, Parkinson's disease and other neurodegenerative diseases. Such a synthetic polypeptides only needs an intravenous injection dose of 3 mg/kg to surprisingly achieve a significant therapeutic effect, comparable to the effect achieved by clinically active standard polypeptide NA1.

The synthetic polypeptide of the present disclosure is easy to synthesize. During the synthesis process, the derivatives of carnosine, anserine or ophidine may be used as synthetic fragments to replace the corresponding two amino acids to further simplify the synthesis process.

## Description of the drawings

Fig. 1: the slice section of brain tissue in the model group.
Fig. 2: the slice section of the brain tissue in group S 1.
Fig. 3: the slice section of the brain tissue in group S3.
Fig. 4: the slice section of the brain tissue in the sham group.
Fig. 5: the results of cerebral infarct area and cerebral infarct inhibition rate, wherein A: cerebral infarct area (infarct Area %), B: cerebral infarct inhibition rate (Inhibition Ratio %); ** indicates $P < 0.01$ compared with the model control group, *** indicates $P < 0.005$ compared with the model control group.
Fig. 6: the NSS score results. The data in the figure is expressed as mean $\pm$ SD, wherein A: NSS score results, B: the reduction rate of the NSS score of each treatment group one day after surgery compared to the model control group; * indicates $P \leq 0.05$ compared with the model control animals.
Fig. 7: the TTC staining results of animals in the model control group.
Fig. 8: the TTC staining results of animals in group S3.
Fig. 9: the TTC staining results of animals in group t-PA.
Fig. 10: the TTC staining results of animals in group S3+tPA.

## Detailed description

In order to make the object, technical solutions and advantages of the present disclosure more clear, the following description is provided in conjunction with the accompanying drawings.

## Example 1: Synthesis of beta-Ala-His-Lys-Leu-Ser-Ser-Ile-Glu-Ser-Asp-Val

1. The Fmoc-Val-CTC resin was obtained by coupling the solid-phase support 2-CTC resin with Fmoc-Val-OH in the presence of an activator system (HoBT, DIC).
2. The Fmoc protecting group of Fmoc-Val-CTC was removed using 20% piperidine followed by washing with DMF.
3. 3 times of excess Fmoc- Asp (OtBu)-OH and 3 times of excess activator were weighted, and a small amount of DMF was added to completely dissolve. The product after dissolution was added to the washed resin, and was washed with DMF after reacting for 1 hour.
4. The second step and the third step were repeated, and the coupling of amino acid with N-terminal Fmoc protection and side-chain protection was sequentially performed from Ser to β-Ala according to the backbone sequence.
5. Cleavage was carried out after the synthesis was completed. The cleavage reagent was formulated as TFA:EDT:Tis:TA:anisole:$H_2O$ of 80:5:1:5:5:4 by volume, and 10 ml cleavage reagent was used per 1g peptide resin. The cleavage was performed at room temperature for about 2 h (120 r/min), followed by precipitation with ice methyl tert-butyl ether. The precipitate at the bottom was the crude product.
6. The crude peptide from the previous step was dissolved and purified with 0.1% TFA/acetonitrile on preparative HPLC.
7. The purified product was lyophilized and the obtained powder was divided and subject to a quality test. The polypeptide beta-Ala-His-Lys-Leu-Ser-Ser-Ile-Glu-Ser-Asp-Val was thus synthesized.

**Example 2: Synthesis of Lys-Leu-Ser-Ser-Ile-Glu-Ser-Asp-Val-beta-Ala-His**

1. The Fmoc-His(Trt)-CTC resin was obtained by coupling the solid-phase support 2-CTC resin with Fmoc-His(Trt)-OH in the presence of an activator system (HoBT, DIC).
2. The Fmoc protecting group of Fmoc-His(Trt)-CTC was removed using 20% piperidine followed by washing with DMF.
3. 3 times of excess Fmoc-beta-Ala-OH and 3 times of excess activator were weighted, and a small amount of DMF was added to completely dissolve. The product after dissolution was added to the washed resin, and was washed with DMF after reacting for 1 hour.
4. The second step and the third step were repeated, and the coupling of amino acid with N-terminal Fmoc protection and side-chain protection was sequentially performed from Val to Lys according to the backbone sequence.
5. Cleavage was carried out after the synthesis was completed. The cleavage reagent was formulated as TFA:EDT:Tis:TA:anisole:$H_2O$ of 80:5:1:5:5:4 by volume , and 10ml cleavage reagent was used per 1g peptide resin. The cleavage was performed at room temperature for about 2 h (120 r/min), followed by precipitation with ice methyl tert-butyl ether. The precipitate at the bottom was the crude product.
6. The crude peptide from the previous step was dissolved and purified with 0.1% TFA/acetonitrile on preparative HPLC.
7. The purified product was lyophilized, and the obtained powder was divided and subject to a quality test. The polypeptide Lys-Leu-Ser-Ser-Ile-Glu-Ser-Asp-Val-beta-Ala-His was thus synthesized.

**Example 3: Synthesis of beta-Ala-His-Lys-Leu-Ser-Ser-Ile-Glu-Ser-Asp-Val-beta-Ala-His**

1. The Fmoc-His(Trt)-CTC resin was obtained by coupling the solid-phase support 2-CTC resin with Fmoc-His(Trt)-OH in the presence of an activator system (HoBT, DIC).
2. The Fmoc protecting group of Fmoc-His(Trt)-CTC was removed using 20% piperidine followed by washing with DMF.
3. 3 times of excess Fmoc-beta-Ala-OH and 3 times of excess activator were weighted, and a small amount of DMF was added to completely dissolve. The product after dissolution was added to the washed resin, and was washed with DMF after reacting for 1 hour.
4. The second step and the third step were repeated, and the coupling of amino acid with N-terminal Fmoc protection and side-chain protection was sequentially performed from Val to beta-Ala according to the backbone sequence.
5. Cleavage was carried out after the synthesis was completed. The cleavage reagent was formulated as TFA:EDT:Tis:TA:anisole:$H_2O$ of 80:5:1:5:5:4 by volume, and 10ml cleavage reagent was used per 1g peptide resin. The cleavage was performed at room temperature for about 2 h (120 r/min), followed by precipitation with ice methyl tert-butyl ether. The precipitate at the bottom was the crude product.
6. The crude peptide from the previous step was dissolved and purified with 0.1% TFA/acetonitrile on preparative HPLC.
7. The purified product was lyophilized, and the obtained powder was divided and subject to a quality test. The polypeptide beta-Ala-His-Lys-Leu-Ser-Ser-Ile-Glu-Ser-Asp-Val-beta-Ala-His was thus synthesized.

**Example 4: The following polypeptides were synthesized using the same process.**

beta-Ala-(1-Methyl-His)-Lys-Leu-Ser-Ser-Ile-Glu-Ser-Asp-Val;
Lys-Leu-Ser-Ser-Ile-Glu-Ser-Asp-Val-beta-Ala-(1-Methyl-His);

beta-Ala-(1-Methyl-His)-Lys-Leu-Ser-Ser-Ile-Glu-Ser-Asp-Val-beta-Ala-(1-Methyl-His);

beta-Ala-(1-Methyl-His)-Lys-Leu-Ser-Ser-Ile-Glu-Ser-Asp-Val;
Lys- Leu-Ser-Ser- Ile-Glu-Ser-Asp- Val-beta-Ala-(1- Methyl-His);

beta-Ala-(1-Methyl-His)-Lys-Leu-Ser-Ser-Ile-Glu-Ser-Asp-Val-beta-Ala-(1-Met hyl-His).

**Example 5: Animal experiments**

Small-animal monitor: VT200 from Rongxianda Technology Co., Ltd., Shenzhen, China.
SD rats: 180-200g, from China National Institutes for Food and Drug Identification.
Suture emboli: M8507 from Maiyue Bio, .
TTC staining solution: R24053 from Yuanye Bio.

Animal model surgery:

The experimental SD rats were weighed and anesthetized with chloral hydrate. After anesthesia, the limbs of the rats were fixed, and the rats were placed in a supine position, connected to the small-animal monitor, and monitored for important physiological parameters such as body temperature, blood pressure and heart rate, etc.. The rats were shaved at neck and disinfected with 75% alcohol cotton ball. A middle incision of about 2 cm length was then made on the neck of the rats, and the submandibular gland of the rats was bluntly separated while avoiding damage to the gland. Then, the left common carotid artery (CCA) of the rats was bluntly separated, and the internal carotid artery (ICA) and external carotid artery (ECA) were carefully separated upward along the CCA. Any damage to vagus nerves was avoided during the separation. The CCA and ICA were clamped respectively with two artery clamps, and the distal end of the ECA was cut off. A silicone suture emboli was inserted into the ECA "port" to the ICA artery clamp. Then the artery clamp was quickly loosened and the tip of the suture emboli was pushed forward through the artery clamp quickly before clamping the ICA again. This step was repeated and the suture emboli was pushed further until the black mark of the suture emboli reached the bifurcation of the ECA and ICA, and the tip of the suture emboli blocked the middle cerebral artery. Then the ECA "port" and the suture emboli were tied tightly using a thread in order to prevent the "escape" of suture emboli or bleeding after the rats waked up. The artery clamps on the CCA and ICA were then loosed and removed to restore the tissue to its original position, and an appropriate amount of penicillin was applied to prevent wound infection. A medical suture needle with thread was used to suture, and the wound was disinfected using povidone-iodine again after the ligation. The experimental rats were monitored with the small-animal monitor to evaluate if their physiological parameters were normal, and the rats were placed on a small-animal electric blanket to keep their body temperature until they waked up and placed in animal cages.

Dosing:

The drugs to be tested were prepared as 3 mg/mL solution. The drugs were administered via tail vein 1-2 h after the ligation.
During the administration, the rats were fastened with a fastening device, and the drugs to be tested were injected into the tail vein of the rats at 3 mg/kg in 1 mL syringe slowly to reduce the cardiopulmonary load of the rats.
The animals were divided into different groups, with 6 rats for each group.

Model group: After the ligation, an equal amount of normal saline was given via tail vein;
Treatment groups S1 (NA1) and S3 (beta-Ala-His-Lys-Leu-Ser-Ser-Ile-Glu-Ser-Asp-Val), administered via tail vein after the ligation;
Sham group: the internal carotid artery (ICA) and external carotid artery (ECA) were separated without the ligation thereafter, and an equal amount of normal saline was administered via tail vein.

Behavioral observation:

The behavioral observation and scoring were performed on each animal 22-24 h after the ligation.
The technicians who did not participate in the above experimental procedure were asked to perform the scoring blindly according to the scoring scale below.

0: walking in a normal straight line;

1: forelimb weakness;

2: hindlimb weakness;

3: mild circling;

4: severe circling;

5: hemiplegia.

TTC staining and quantitative analysis:

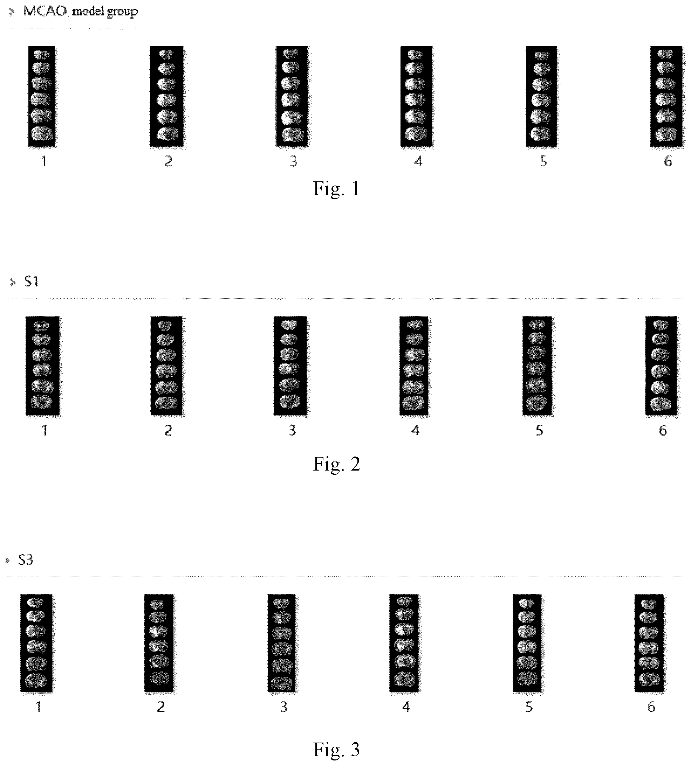

After the behavioral observation, rats were euthanized and their brains were removed. Each brain tissue was transversely cut into 6 slices with a 2mm thickness, then transferred to TTC staining solution, incubated in a 37°C incubator without light for 10min, and taken pictures (see Figs. 1-4). The TTC-stained brain tissues and the remaining non-stained brain tissues were stored at -20°C.

The quantitative analysis of photos after TTC staining was performed using Image J software.

Cerebral infarct volume %=(total infarct area * slice thickness)/(total brain slice area*slice thickness) * 100%.

Results:

1. The TTC quantification results are shown in Table 1:

**Table 1**

| No. | 1 | 2 | 3 | 4 | 5 | 6 | Mean value |
| --- | --- | --- | --- | --- | --- | --- | --- |
| model group | 0.407 | 0.402 | 0.392 | 0.420 | 0.377 | 0.340 | 0.390 |
| S1(NA1) | 0.187 | 0.218 | 0.153 | 0.145 | 0.107 | 0.134 | 0.157 |
| S3 | 0.128 | 0.186 | 0.075 | 0.158 | 0.210 | 0.146 | 0.128 |
| Sham group | 0.048 | 0.038 | 0.042 | 0.051 | 0.039 | 0.044 | 0.044 |

As shown in Table 1, the rats with cerebral ischemia injected with normal saline had an infarct volume of $0.390\pm0.028$; the rats with cerebral ischemia injected with 3mg/kg Ala-His-Lys-Leu-Ser-Ser-Ile-Glu-Ser-Asp-Val (S3) had an infarct volume of $0.150\pm0.047$. As a control, the Sham group had an infarct volume of $0.044\pm0.005$. Compared with the model group, S3 showed significant biological activities and the results of S3 were not statistically different from S1. NA1 (nerinetide) is a neuroprotective agent (Michael Tymianski and Jonathan D. Garman. Model systems and treatment regimes for treatment of neurological disease. 2015, US Patent, US8940699B2), which can interfere with postsynaptic density protein 95 (PSD-95) by terminating the production of intracellular NO free radicals. In preclinical ischemic stroke model, NA1 can reduce the infarct area of experimental cerebral ischemia-reperfusion in animals (cynomolgus monkeys), and improve the functional prognosis, therefore it is a good positive control. Dr. Hill assessed the efficacy and safety of novel neuropeptide NA-1 via intravenous injection (2.6 mg/kg) in human ischaemia-reperfusion that occurs with rapid endovascular thrombectomy in patients who had an acute ischemic stroke (AIS), showing that the treatment with NA1 improved outcomes. (Michael D Hill et al. Efficacy and safety of nerinetide for the treatment of acute ischaemic stroke (ESCAPE-NA1): a multicentre, double-blind, randomised controlled trial. Lancet. Published online February 20, 2020).

2. The behavioral scoring results are shown in Table 2:

**Table 2**

| No. | 1 | 2 | 3 | 4 | 5 | 6 | Mean value | Standard deviation |
| --- | --- | --- | --- | --- | --- | --- | --- | --- |
| model group | 4 | 4 | 3 | 5 | 4 | 3 | 3.8 | 0.8 |
| S1 | 2 | 2 | 2 | 2 | 1 | 3 | 2.0 | 0.6 |
| S3 | 3 | 4 | 1 | 2 | 3 | 2 | 2.5 | 1.0 |
| Sham group | 0 | 0 | 0 | 0 | 0 | 0 | 0.0 | 0.0 |

As shown in Table 2, the synthetic peptides provided herein have physiological activities. The rats with cerebral ischemia injected with normal saline had a behavioral score of 3.8±0.8; the rats with cerebral ischemia injected with 3mg/kg beta-Ala-His-Lys-Leu-Ser-Ser-Ile-Glu-Ser-Asp-Val (S3) had a behavioral score of 2.5±1.0; and as a comparison, the rats with cerebral ischemia given 3 mg/kg NA1 intravenously had a behavioral score of 2.0±0.6. As a control, the Sham group had a behavioral score of 0.0±0.0. Compared with the model group, S3 showed significant biological activities, and the results of S3 were not statistically different from S1.

**Example 6: Combination administration of S3 (beta-Ala-His-Lys-Leu-Ser-Ser-Ile-Glu-Ser-Asp-Val) and the stroke drug t-PA**

1. The preparation of products to be tested: t-PA was the recombinant human TNK tissue-type plasminogen activator for injection (Mingfule) purchased from Guangzhou Mingkang Bioengineering Co., Ltd., administered at $1.0 \times 10E7$ IU/16mg/rat. Taking the animal weight 300 g as an example, the preparation method is shown in Table 3.

**Table 3**

| Group | Dose (mg/kg) | Concentration (mg/mL) | Preparation |
|---|---|---|---|
| S3 group | 3 | 1 | Weigh 9 mg of S3 and add it to 9 mL of sterile water for injection to obtain a 1 mg/mL solution. |
| t-PA group | 3 | 1 | Add 10 mL of sterile water for injection into a bottle of t-PA solution to obtain a 2 mg/mL stock solution. Add 4.5 mL of the stock solution into 10.5 mL of sterile water for injection to obtain a 1 mg/mL solution. |
| S3+t-PA group | 3(S3)+3 (t-PA) | 1(S3)+1(t-PA) | Same as for S3 and t-PA. |

The preparation was performed aseptically in a biological safety cabinet, and the consumables used were also sterilized.
The prepared S3 was stored in an ice box without light, and returned to normal temperature before injection. The prepared t-PA was temporarily stored at normal temperature.

2. Animal strain/grade

Strain: Sprague-Dawley (SD) rats;
Grade: SPF grade;
Number and gender of animals entering the adaptation period: 40, male;
Number and gender of animals used: 36, male;
Animal age and weight
Body weight: 151.78 - 186.9 g when entering the adaptation period, 242.95 - 289.31 g when grouping;
Age: about 5-7 weeks old when entering the adaptation period, and 6-8 weeks old when grouping;
Source: SPF (Beijing) Biotechnology Co., Ltd., Beijing, China;
Environmental adaptation: Animals were adapted to the environment for 5 days after received, the main inspection contents during the adaptation period included whether the animals were consistent with the quality indicators required when ordering, their general condition, and whether their weight reaches the weight range required by the test, and unqualified animals were excluded from the experiment;
Animal feeding: plastic rat cage (L × W × H: 46.6 cm × 30 cm × 21.5 cm); 5 rat/cage during the environmental adaptation period, and 3-4 rat/cage during the test;
The condition standard for breeding environment: Chinese National Standard GB14925-2010;
The environment control system: WINCC7.3 EMS series of environmental monitoring system;
Temperature: room temperature 20-26 °C (daily temperature difference ≤ 4 °C);
Relative humidity: 40-70%;
Lighting: artificial lighting, 12/12 hrs alternating light and dark;
Air exchange: no less than 15 times per hour;

Feed: Rat and mouse breeding feed, batch No. 21103213, purchased from Beijing Keao Xieli Feed Co., Ltd..

No abnormalities were observed in the general condition of the animals during the adaptation period. After the adaptation period, the animal weight were 185.46-230.82g, which did not reach the weight required for animal modeling, so the animal adaptation period was extended to 12 days. After the adaptation period, animals weighing 240-290 g were selected for dividing into groups for modeling. 24 hours after administration, the animal numbered 2M006 in the S3 group was found dead. An autopsy revealed that the animal numbered 1M005 in the model control group had brain tissue deficiency and was determined to have abnormal brain development. In addition, subarachnoid hemorrhage was found in the anatomy of the animals numbered 2M001 in the S3 group and 4M002 in the S3+t-PA group. The cerebral infarct area of the rat numbered 4M009 in the S3+t-PA group was 25.19%, which was greater than the mean ± 3 standard deviations of the remaining animals in this group and was determined to be an outlier. The data of the above animals was not included in the final statistical analysis. The data of the remaining animals in each group was included in the final statistical analysis. The number of animals included in the final statistical analysis was 8 animals for the model control group, 7 animals for S3 group, 9 animals for the t-PA group and 7 animals for S3+t-PA group.

3. Modeling

1) Anesthesia induction: the rats were placed in an anesthesia induction box filled with 3.0% isoflurane for anesthesia induction.
2) Immobilization: Anesthesia-induced animals were transferred to the operating table, and anesthesia was maintained with 2.0%-2.5% isoflurane at 200 mL/min using a small-animal gas anesthesia machine, and the eyelid reflex and pain response of the rats were observed. The surgery can be started only after the eyelid reflex response and the pain response on limbs and tail disappeared;
3) Cerebral ischemia reperfusion surgery:

A. Isolating and exposing blood vessels: the skin of the surgical area was shaved, and the rats were placed under a surgical microscope. The skin of the rat was cut along the midline with ophthalmic scissors, with a cut of about 2 cm length. Through the right neck of the rat, the muscle tissue of the right neck was bluntly isolated and opened with a microforcep to expose the right common carotid artery (CCA), and then isolated upward along the common carotid artery to further expose the external carotid artery (ECA) and internal carotid artery (ICA);
B. The proximal end of CCA was ligated and the CCA at the Y-shaped branch was temporarily clamped; the ligated site of CCA to the clamped site of ICA were threaded, tied with a loose knot (pre-ligation) for later use, and a small incision at the proximal end of the pre-ligation was made;
C. Inserting the suture emboli: Suture emboli No. 4-0 was inserted through the incision of CCA, pushed slowly and gently into the internal carotid artery, and stopped temporarily at the artery clamp of ICA. The pre-ligation thread was tied tightly (to avoid severe bleeding when pushing the suture emboli), then the artery clamp that blocked the blood flow of ICA was removed, and the suture emboli was immediately pushed into ICA until entering into the cranium. One should be careful not to insert the suture emboli into the pterygopalatine artery, which is another branch of the internal carotid artery and is one of the extra-cranial branches of ICA. When the suture emboli entered the pterygopalatine artery to a depth of about 10 mm, it cannot be inserted further, and then one should slightly draw back the suture emboli, adjust the direction, and insert again;
D. Fixing the suture emboli and stitching up the incision: if one felt slight resistance when the suture emboli was inserted about 18 mm depth from the bifurcation of the common carotid artery, it means that the tip of the suture emboli has entered into the cerebral anterior artery (ACA), and the side wall of the suture emboli has blocked the opening of the middle cerebral artery. Then the insertion was terminated and the time was recorded. The artery clamp on CCA was removed, and the incision was closed after there was no active bleeding observed;
E. Performing cerebral reperfusion: the ischemic rats were placed at room temperature, and the anesthesia was induced 120 mins thereafter. The suture emboli was slowly and lightly drawn to pull the tip of suture emboli back into the common carotid artery while maintaining anesthesia, in order to perform the middle cerebral artery reperfusion;
F. Disinfecting the incision with povidone-iodine.

4. Animal grouping

Group design: 4 groups in total, namely model control group, S3 group, t-PA group and S3+t-PA group;
Number of animals: 9 animals per group, 36 animals in total;
Sex ratio: according to what has been reported by JW Simpkins and RL Roof , estrogen and progesterone have neuroprotective effects on ischemic cerebral infarct in adult rats, and in order to exclude the influence of estrogen and progesterone on experimental results, all the animals in this experiment were male;
Grouping method: random grouping was performed according to the latest body weight of rats before grouping;

See Table 4 below for specific grouping information.

**Table 4**

| Group | Drug | Dose (mg/kg) | concentration (mg/mL) | volume (mL/kg) | Administration route | Animal number |
|---|---|---|---|---|---|---|
| model control group | Vehicle | - | - | 6 | i.v. | 1M001-1M008 |
| S3 group | S3 | 3 | 1 | 3+3[a] | i.v. | 2M001-2M008 |
| t-PA group | t-PA | 3 | 1 | 3+3[b] | i.v. | 3M001-3M008 |
| S3+ t-PA group | S3+t-PA | 3+3 | 1+1 | 3+3[c] | i.v. | 4M001-4M008 |

Note: The first digit of the animal number represents the group (1, 2, 3 and 4 represent the model control group, S3 group, t-PA group and S3+t-PA group respectively), and the second letter represents the gender (M is male), the last three digits represent the animal serial number. a means giving 3 mL S3 and 3 mL sterile water for injection, b means giving 3 mL t-PA and 3 mL sterile water for injection, c means giving 3 mL S3 and 3 mL t-PA.

Route of administration: tail vein injection;
Administration time: immediately after ischemia-reperfusion (within 5 minutes);
Dosing frequency and cycle: 1 dose;

The day of modeling was defined as D0, and the day before was defined as D-1.

5. Observation and measurement

General status observation

Animals observed: all the surviving animals planned to be observed;
Observation time: once a day, and the frequency can be increased if the animal starts to behave abnormal;
What were observed: including but not limited to general manifestations, behavioral status, eye, mouth, nose and mouth area, ear, hair and skin, feces, urine, genital organ and other toxic symptoms. A detailed description was required if there was any abnormality.

Body Weight

Animals measured: all the surviving experimental animals planned to be measured;
Measurement time: the body weight was measured at least twice during the adaptation period, once 24 hours before surgery for grouping, and once 24 hours after surgery.

Behavioral assessments

**Bederson score**

Measurement time: 50-60 mins after ischemia on the day of modeling, in order to evaluate whether the ischemia was preformed successfully;

Animals measured: all the animals survived in surgery;
Measurement method: the Bederson scoring standard was used to score animals;

**Table 5: Bederson Scoring Standard for Rats**

| Score | Scoring standard |
|---|---|
| 0 | no symptom of nerve damage |
| 1 | The rat was lifted from the tail, and the forelimb on the side opposite to the surgery happened was flexed |
| 2 | The rat was placed on a smooth surface, and its shoulder on the opposite side of the infarct was pushed by hand, and the resistance decreased |
| 3 | The animal fell to the opposite side of the infarct or walked in circles when walking freely |
| 4 | Paraplegia, no spontaneous movement of limbs |

The rats with a score of greater than or equal to 1 were successfully modeled, and the rats that failed the modeling were excluded.

**NSS scoring of rats**

Measurement time: 24 hours before the modeling, 24 and 72 hours after the modeling;
Animals measured: all the surviving animals planned to be measured;
Measurement method: a motor function test, sensory test, balance test, and reflex and abnormal behavior test were performed on animals, according to the rat for details.

**Table 6: Rat Neurological Severity Scores (NSS)**

| Classification | Score |
|---|---|
| **Motor function test (6)** | |
| Lift the rat by its tail (3) | |
| Forelimb flexion | 1 |
| Hindlimb flexion | 1 |
| Head tilted more than 10 degrees within 30 seconds | 1 |
| Put the rat on the platform (normal 0, maximum 3) | |
| Walk normally | 0 |
| Unable to crawl straight | 1 |
| Walk in circles around the hemiplegic side | 2 |
| Fall to the hemiplegic side | 3 |
| **Sensory test (2)** | |
| Placement experiment (visual and tactile test) | 1 |
| Proprioception testing (deep sensation, the affected limb was placed on edge of table and observed for limb contraction response) | 1 |
| **Balance test (6)** | |
| Grab one side of the rod | 1 |
| Hold onto the rod, with a limb drops | 2 |
| Hold onto the rod with both limbs drop or hold onto the rod and rotate for >60 seconds | 3 |
| Try to balance but fail and fall (>40 seconds) | 4 |
| Try to balance but fail and fall (>20 seconds) | 5 |
| Fall (<20 seconds) | 6 |
| **Reflex and abnormal behavior test (4)** | |
| Pinna reflection | 1 |

(continued)

| Reflex and abnormal behavior test (4) | |
|---|---|
| Corneal reflection | 1 |
| Startle reflection | 1 |
| Convulsion, myoclonus, dystonia | 1 |

Anatomy and pathology examination

Dissection time: D1 (equivalent to 24 hours after modeling, and the day of modeling is defined as D0);
Dissected animals: all surviving animals included in the study;
Anesthesia and euthanasia: 3% pentobarbital sodium was applied for intraperitoneal anesthesia with an injection dose of 60 mg/kg. After anesthesia, the rats were euthanatized by bleeding from the abdominal aorta.

**Pathological (TTC staining) assessment**

The animals being tested: all the living animals;
Examination method: the animals were euthanized, the brains were quickly removed and placed in a -20°C refrigerator until the brain tissues became hard. Then the rat brains were taken out and cut into 2 mm thick slices, and a total of 6-8 slices from the olfactory bulb to the back were placed in a six-well plate filled with 0.5% TTC solution (prepared in PBS), incubated in a 37°C incubator without light for 20 min, and then stored in 10% formaldehyde solution without light.

The normal tissues were stained rose red, and infarcted tissues were white. Each brain section was placed in order on a filter paper, and taken pictures using digital camera, and the white tissues were carefully removed and weighed. The infarct area (%) was calculated as the percentage of the infarct tissue weight to the whole brain weight, or calculated using image processing means. The inhibition rate (%) of each treatment group was calculated based on the infarct area, using the following formula:

$$\text{Inhibition rate (\%)} = \frac{\text{the infarct area of model group - the infarct area of treatment group}}{\text{the infarct area of model group}} \times 100\%$$

Statistical analysis:

The measurement results were expressed as mean $\pm$ standard deviation. The data from a group will not be included in the statistical comparison if the sample number of the group was less than 3.
The data were input and statistically analyzed by Excel 2010, GraphPad Prism 7, SPSS 22.0 and Stata 15.0 software. Firstly, the measurement was subject to LEVENE' test for homogeneity of variance. When the variance was homogeneous ($p>0.05$), the results from the variance analysis was directly used to determine whether the overall difference was statistically significant. When the overall difference was statistically significant ($p<0.05$), the Dunnett-t test was used to compare the difference between groups. When the overall difference was not statistically significant ($p>\_0.05$), the statistical analysis was ended. When the LEVENE variance was not homogeneous ($p\leq0.05$), the non-parametric test (Kruskal-Wallis H test) was used. When the Kruskal-Wallis H test showed that the overall difference was statistically significant ($p<0.05$), the Mann-Whitney U test was used to compare the difference between groups. When the Kruskal-Wallis H test showed that the overall difference was not statistically significant ($p\geq0.05$), the statistical analysis was ended.

**6. Results**

The effects of S3 and S3+t-PA on cerebral infarct area in model animals:

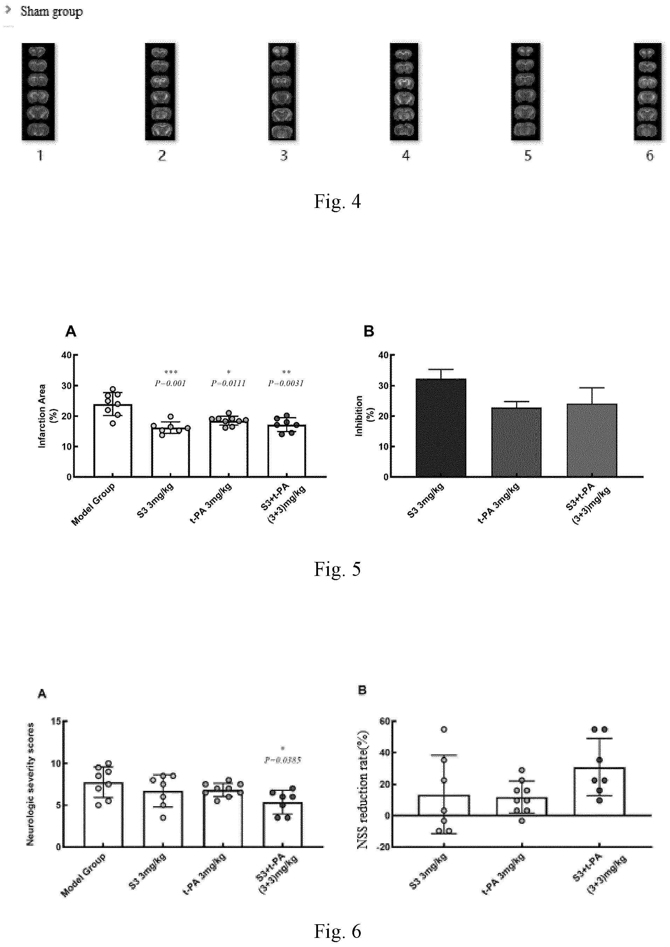

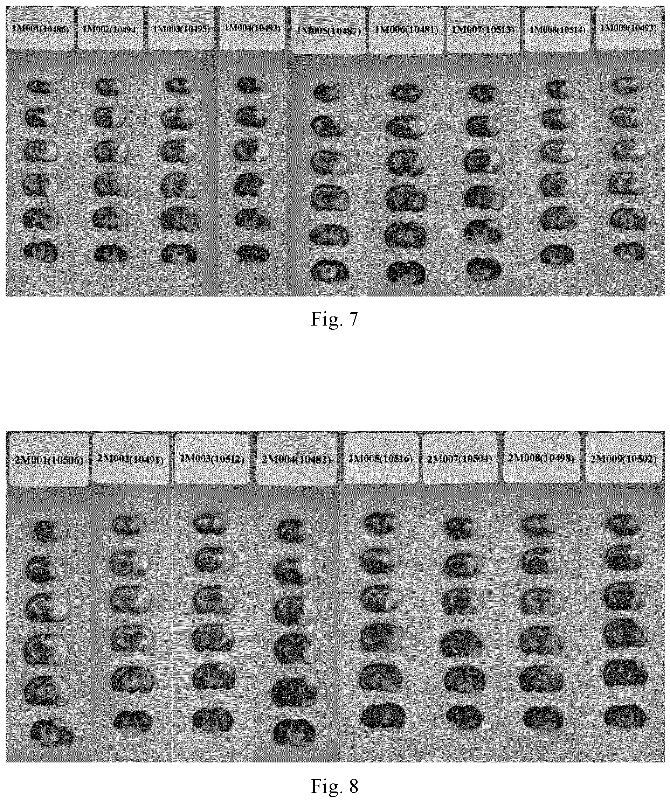

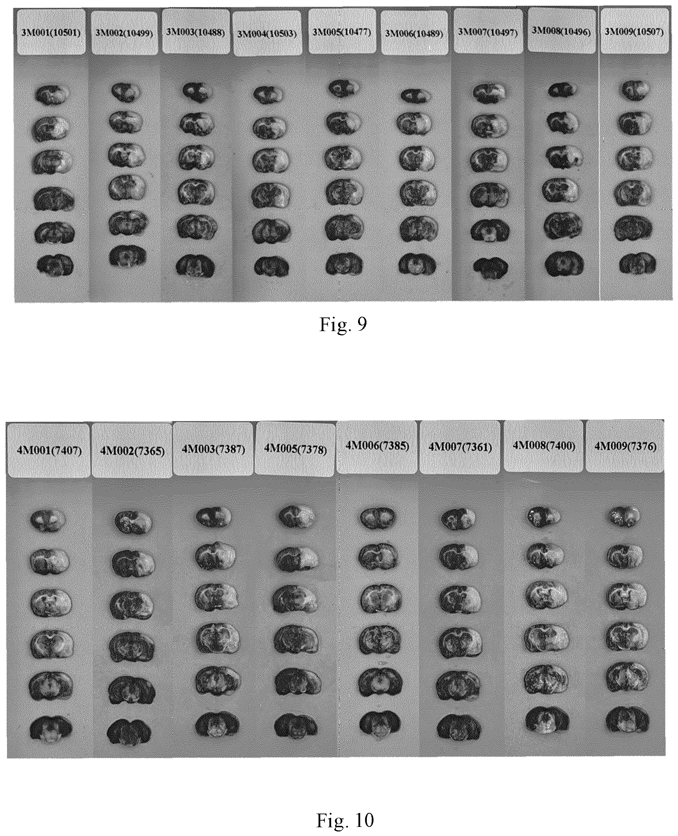

The main purpose of the treatment of acute ischemic stroke is to minimize the degree and area of neuronal cell damage caused by ischemia. In this trial, drug intervention was performed immediately after ischemia-reperfusion, and TTC staining was performed on model animal brains after 24 hours. By measuring the infarct area of each group and calculating the inhibition rate, the anti-ischemic stroke pharmacodynamics of S3 and combination of S3 with t-PA were evaluated. The cerebral infarct area and cerebral infarct inhibition rate of animals were shown in Table 7 and Figures 5 and 7-10.

24 hours after the surgery, the cerebral infarct area of the model control group was $21.493 \pm 2.734\%$. The cerebral infarct areas of animals in S3 group, t-PA group and S3+t-PA group were $16.248 \pm 1.749\%$, $18.522 \pm 1.372\%$ and $17.203 \pm 2.098\%$ respectively, which were significantly reduced compared with the model control group ($P \leq 0.05$). The cerebral infarct inhibition rates of animals in S3 group, t-PA group and S3+t-PA group were $32.279 \pm 7.291\%$, $22.802 \pm 5.719\%$ and $28.301 \pm 8.746\%$ respectively. The above results suggest that under the conditions of this experiment, S3 group, t-PA group and S3+t-PA group all have significant inhibitory effects on cerebral infarct in ischemic stroke rats. The Q value of the combination of S3 and t-PA was calculated and estimated based on the average cerebral infarct inhibition rate of each group, showing a Q value of 0.593 and indicating that 3 mg/kg S3 and 3 mg/kg t-PA had no significant synergistic effect on the inhibition of cerebral infarct under the experimental condition.

**Table 7:** The cerebral infarct area and the inhibition rate of cerebral infarct in each group

| Group | Whole brain weight (g) | Infarct area weight (g) | infarct area (%) | Cerebral infarct inhibition rate (%) | Q value |
|---|---|---|---|---|---|
| Model control group (N=8) | $1.298 \pm 0.094$ | $0.31 \pm 0.044$ | $23.993 \pm 3.531$ | - | |
| Group S3 (N=7) | $1.435 \pm 0.03$ | $0.233 \pm 0.024$ | $16.248 \pm 1.749^{***}$ | $32.279 \pm 7.291$ | |
| t-PA group (N=9) | $1.406 \pm 0.058$ | $0.26 \pm 0.022$ | $18.522 \pm 1.372^{*}$ | $22.802 \pm 5.719$ | 0.593 |
| S3+t-PA group (N=7) | $1.378 \pm 0.099$ | $0.236 \pm 0.027$ | $17.203 \pm 2.098^{**}$ | $28.301 \pm 8.746$ | |

Note: The data in the table is expressed as mean $\pm$ standard deviation (Mean $\pm$ SD); "N" represents the number of animals in each group for statistical analysis, "-" indicates no data available, ** indicates P<0.01 compared to the model control group, *** indicates P<0.005 compared to the model control group; Q value is determined by the average cerebral infarct inhibition rate of S3+t-PA group/(the average cerebral infarct inhibition rate of S3 group + the average cerebral infarct inhibition rate of t-PA group - the average cerebral infarct inhibition rate of S3 group x the average cerebral infarct inhibition rate of t-PA group). Q value of Q<0.85 indicates antagonism, $0.85 \leq Q < 1.15$ indicates additive effect, and $Q \geq 1.15$ indicates synergistic effect.

The effect of S3 and S3+t-PA on neurobehavioral scores of model animals:

The main purpose of clinical treatment for cerebral infarct is to restore the patient's neurological function as much as possible and to improve the quality of life after stroke. In this experiment, the animals were subject to Bederson scoring 1 hour after ischemia to assess the ischemia in animals. 24 hours after modeling, the neurobehavioral function of living animals was evaluated with NSS scoring scale in order to evaluate the effects of S3 and S3+t-PA in improving the neurological function of animals. The Bederson scoring results of animals were shown in Tables 8 and 10, and the NSS scoring results were shown in Tables 9 and 10 and Figure 6.

One hour after ischemia, the Bederson score of all the model animals was 3, suggesting that all the model animals were successfully ischemic.

One day after the surgery, the NSS score of the animals in the ischemia-reperfusion model control group was $7.75 \pm 1.71$, and the NSS scores of the animals in S3 group, t-PA group and S3+t-PA group were $6.71 \pm 1.79$, $6.83 \pm 0.75$ and $5.36 \pm 1.30$ respectively, among which the NSS of the animals in S3+t-PA group was significantly reduced compared with the model control group (P=0.0385). The NSS score reduction rate of each treatment group relative to the model control group was determined, S3 group, t-PA group and S3+t-PA group being $13.36 \pm 23.1\%$, $11.83 \pm 9.62\%$ and $30.88 \pm 16.79\%$ respectively. The Q value of the combination of S3 and t-PA calculated based on the NSS reduction rates was 1.308, indicating that S3 and t-PA have a synergistic effect on improving NSS score. The above results indicate that under the condition of this experiment, the combination of S3 with t-PA led to a significant improvement on neurobehavior of stroke rats, and S3 and t-PA had a synergistic effect in the improvement of neurological damage in ischemia-reperfusion mice.

**Table 8:** Animal Bederson Scoring Results

| Group | Bederson |
|-------|----------|
| Model control group (N=8) | 3±0 |
| S3 group (N=7) | 3±0 |
| t-PA group (N=9) | 3±0 |
| S3+t-PA group (N=7) | 3±0 |

Note: The data in the table are expressed as mean ± standard deviation (Mean ± SD); "N" represents the number of animals in each group for statistical analysis.

**Table 9:** Animal NSS Scoring Results

| Group | NSS | | NSS reduction rate (%) | Q value |
|-------|-----|-----|------------------------|---------|
| | D0 | D2 | | |
| Model control group (N=8) | 0.00±0.00 | 7.75±1.71 | - | |
| S3 group (N=7) | 0.00±0.00 | 6.71±1.79 | 13.36±23.1 | 1.308 |
| t-PA group (N=9) | 0.00±0.00 | 6.83±0.75 | 11.83±9.62 | |
| S3+t-PA group (N=7) | 0.00±0.00 | 5.36±1.30* | 30.88±16.79 | |

Note: The data in the table are expressed as mean ± standard deviation (Mean ± SD); "N" represents the number of animals in each group for statistical analysis; * indicates P<0.05 compared to the model control group; Q value is determined by the average NSS reduction rate of S3+t-PA group/(the average NSS reduction rate of S3 group + the average NSS reduction rate of t-PA group - the average NSS reduction rate of S3 group x the average NSS reduction rate of t-PA group), Q value of Q<0.85 indicates antagonism, 0.85<Q<1.15 indicates additive effect, and Q>_1.15 indicates synergistic effect.

The effect of S3 and S3+t-PA on the body weight of model animals:

Cerebral ischemia-reperfusion is an acute injury model, wherein the weight of experimental animals will drop dramatically after MCAO modeling. Thus, the weight of experimental animals is one of the most basic sensitive parameters to comprehensively reflect the health status of animals. In this experiment, the body weight of all the animals was monitored on the day before the surgery and on the first day after the surgery, and the changes of animal's body weight were observed and recorded. The experiment showed that there was no significant difference in the body weight of animals in each group before modeling and 24 hours after modeling, indicating no significant impact in each group on the animal weight change under the experimental condition.

General status observation results:

After the MCAO modeling, all the animals in the model control group and each treatment group exhibited symptoms such as unsteady gait, drooling, and turning in circles and there was no difference between groups; 24 hours after the surgery, most of the surviving animals showed abnormal symptoms associated with stroke, such as unsteady gait, turning in circles, hair standing, drooling and dirty nose, eyes, and mouth, etc. There was no significant difference in the types and severity of abnormal symptoms between groups.

In summary, the present experiment applied the classic suture emboli method to establish an MCAO ischemia-reperfusion model for the purpose of evaluating the anti-stroke effects of S3 and S3 combined with t-PA and exploring whether there is a synergistic effect between S3 and t-PA. The model control group and each treatment group were administered once immediately after ischemia-reperfusion (within 5 minutes). One day after the surgery, the neurological damage was evaluated according to the NSS score, and the animals were dissected and the brain tissues were stained with TTC to determine the cerebral infarct area. Based on the cerebral infarct area and the improvement of neurological function, the neuroprotective effects of S3 and S3 combined with t-PA on ischemic stroke in rats were evaluated. The results indicated that:

A) S3 and S3 combined with t-PA have significant inhibitory effects on cerebral infarct in stroke rats. One day after the surgery, the cerebral infarct areas of animals in S3 group, t-PA group and S3+t-PA group were all significantly reduced compared with the model control group (P≤0.05). The cerebral infarct inhibition rates of the animals in S3 group, t-PA group and S3+t-PA group were 32.279±7.291%, 22.802±5.719% and 28.301±8.746%, respectively. The results indicate that S3 group, t-PA group and S3+t-PA group all have significant inhibitory effects on cerebral infarct in ischemic stroke rats.

B) S3 combined with t-PA has a significant inhibitory effect on neurological damage in stroke rats. 24 h after the surgery, the NSS scores of the animals in S3 group, t-PA group and S3+t-PA group were reduced to different degrees compared with the model control group, among which the NSS of the animals in S3+t-PA group was significantly reduced compared with the model control group (P=0.0385). The NSS score reduction percentages of the animals in each treatment group compared with the model control group were 13.36±23.1%, 11.83±9.62% and 30.88±16.79% respectively. The results suggest that under the condition of this experiment, S3 combined with t-PA can significantly improve the neurological damage of rats with hemorrhagic stroke.

C) S3 and t-PA have a synergistic effect on improving neurological damage in stroke rats. In this experiment, the Q values of S3 combined with t-PA in terms of the NSS score and the cerebral infarct inhibition rate calculated based on the neurological function reduction rate and the cerebral infarct inhibition rate were 1.308 and 0.593 respectively, among which the Q value of the NSS score is greater than 1.15, indicating that S3 and t-PA have a synergistic effect in improving neurological damage in stroke rats. There was no obvious synergistic effect in inhibiting cerebral infarct under the condition of this experiment.

In conclusion, the results of the present experiment showed that S3, t-PA and the combination of S3 with t-PA have significant therapeutic effects on acute ischemic stroke, and the combination of S3 and t-PA has a synergistic effect on improving the neurobehavior of rats with acute ischemic stroke.

**Table 10:** Summary of individual neurobehavioral scores

| Group | Animal number | System number | Bederson Scores | NSS D0 | NSS D2 | NSS reduction rate (%) |
|---|---|---|---|---|---|---|
| model control group | 1M001 | 10486 | 3 | 0 | 9 | |
| | 1M002 | 10494 | 3 | 0 | 9.5 | |
| | 1M003 | 10495 | 3 | 0 | 10 | |
| | 1M004 | 10483 | 3 | 0 | 7.5 | |
| | 1M006 | 10481 | 3 | 0 | 5 | |
| | 1M007 | 10513 | 3 | 0 | 7 | |
| | 1M008 | 10514 | 3 | 0 | 8.5 | |
| | 1M009 | 10493 | 3 | 0 | 5.5 | |
| S3 group | 2M002 | 10491 | 3 | 0 | 7.5 | 3.23 |
| | 2M003 | 10512 | 3 | 0 | 3.5 | 54.84 |
| | 2M004 | 10482 | 3 | 0 | 8 | -3.23 |
| | 2M005 | 10516 | 3 | 0 | 6 | 22.58 |
| | 2M007 | 10504 | 3 | 0 | 5 | 35.48 |
| | 2M008 | 10498 | 3 | 0 | 8.5 | -9.68 |
| | 2M009 | 10502 | 3 | 0 | 8.5 | -9.68 |
| t-PA group | 3M001 | 10501 | 3 | 0 | 7 | 9.68 |
| | 3M002 | 10499 | 3 | 0 | 8 | -3.23 |
| | 3M003 | 10488 | 3 | 0 | 7.5 | 3.23 |
| | 3M004 | 10503 | 3 | 0 | 6.5 | 16.13 |
| | 3M005 | 10477 | 3 | 0 | 6.5 | 16.13 |
| | 3M006 | 10489 | 3 | 0 | 7.5 | 3.23 |
| | 3M007 | 10497 | 3 | 0 | 6 | 22.58 |
| | 3M008 | 10496 | 3 | 0 | 5.5 | 29.03 |
| | 3M009 | 10507 | 3 | 0 | 7 | 9.68 |

(continued)

| Group | Animal number | System number | Bederson Scores | NSS | | NSS reduction rate (%) |
|---|---|---|---|---|---|---|
| | | | | D0 | D2 | |
| S3+t-PA group | 4M001 | 10505 | 3 | 0 | 5 | 35.48 |
| | 4M003 | 10508 | 3 | 0 | 7 | 9.68 |
| | 4M004 | 10511 | 3 | 0 | 3.5 | 54.84 |
| | 4M005 | 10509 | 3 | 0 | 6.5 | 16.13 |
| | 4M006 | 10478 | 3 | 0 | 3.5 | 54.84 |
| | 4M007 | 10484 | 3 | 0 | 6 | 22.58 |
| | 4M008 | 10479 | 3 | 0 | 6 | 22.58 |
| | 4M001 | 10505 | 3 | 0 | 5 | 35.48 |

**Table 11:** Summary of individual cerebral infarct area

| Group | Animal number | System number | Left brain weight (g) | Right brain weight (g) | Whole brain weight (g) | Weight of infarct area (g) | Infarct area (%) | Cerebral infarct inhibition rate (%) |
|---|---|---|---|---|---|---|---|---|
| model control group | 1M001 | 10486 | 0.530 | 0.575 | 1.105 | 0.296 | 26.787 | - |
| | 1M002 | 10494 | 0.577 | 0.679 | 1.256 | 0.363 | 28.901 | - |
| | 1M003 | 10495 | 0.643 | 0.741 | 1.384 | 0.346 | 25.000 | - |
| | 1M004 | 10483 | 0.614 | 0.689 | 1.303 | 0.313 | 24.021 | - |
| | 1M006 | 10481 | 0.664 | 0.736 | 1.400 | 0.247 | 17.643 | - |
| | 1M007 | 10513 | 0.653 | 0.715 | 1.368 | 0.301 | 22.003 | - |
| | 1M008 | 10514 | 0.628 | 0.724 | 1.352 | 0.367 | 27.145 | - |
| | 1M009 | 10493 | 0.601 | 0.617 | 1.218 | 0.249 | 20.443 | - |
| S3 group | 2M002 | 10491 | 0.681 | 0.707 | 1.388 | 0.223 | 16.066 | 33.038 |
| | 2M003 | 10512 | 0.681 | 0.731 | 1.412 | 0.217 | 15.368 | 35.947 |
| | 2M004 | 10482 | 0.669 | 0.744 | 1.413 | 0.281 | 19.887 | 17.114 |
| | 2M005 | 10516 | 0.705 | 0.752 | 1.457 | 0.223 | 15.305 | 36.209 |
| | 2M007 | 10504 | 0.683 | 0.768 | 1.451 | 0.200 | 13.784 | 42.552 |
| | 2M008 | 10498 | 0.691 | 0.793 | 1.484 | 0.250 | 16.846 | 29.786 |
| | 2M009 | 10502 | 0.677 | 0.761 | 1.438 | 0.237 | 16.481 | 31.308 |
| t-PA group | 3M001 | 10501 | 0.735 | 0.791 | 1.526 | 0.291 | 19.069 | 20.521 |
| | 3M002 | 10499 | 0.696 | 0.761 | 1.457 | 0.268 | 18.394 | 23.336 |
| | 3M003 | 10488 | 0.636 | 0.748 | 1.384 | 0.229 | 16.546 | 31.037 |
| | 3M004 | 10503 | 0.640 | 0.732 | 1.372 | 0.289 | 21.064 | 12.207 |
| | 3M005 | 10477 | 0.686 | 0.731 | 1.417 | 0.272 | 19.195 | 19.996 |
| | 3M006 | 10489 | 0.661 | 0.728 | 1.389 | 0.260 | 18.719 | 21.984 |
| | 3M007 | 10497 | 0.667 | 0.752 | 1.419 | 0.230 | 16.209 | 32.445 |
| | 3M008 | 10496 | 0.566 | 0.736 | 1.302 | 0.239 | 18.356 | 23.493 |
| | 3M009 | 10507 | 0.641 | 0.743 | 1.384 | 0.265 | 19.147 | 20.196 |
| S3+PA group | 4M001 | 10505 | 0.717 | 0.832 | 1.549 | 0.218 | 14.074 | 41.343 |
| | 4M003 | 10508 | 0.639 | 0.716 | 1.355 | 0.232 | 17.122 | 28.639 |
| | 4M004 | 10511 | 0.653 | 0.770 | 1.423 | 0.207 | 14.547 | 39.371 |
| | 4M005 | 10509 | 0.656 | 0.731 | 1.387 | 0.267 | 19.250 | 19.768 |
| | 4M006 | 10478 | 0.602 | 0.600 | 1.202 | 0.217 | 18.053 | 24.756 |
| | 4M007 | 10484 | 0.665 | 0.755 | 1.420 | 0.287 | 20.211 | 15.762 |
| | 4M008 | 10479 | 0.636 | 0.675 | 1.311 | 0.225 | 17.162 | 28.469 |

**Table 12:** Summary animal body weights (g)

| Group | Animal number | System number | Weight(g) | |
|---|---|---|---|---|
| | | | D0 | D2 |
| model control group | 1M001 | 10486 | 281.17 | 212.52 |
| | 1M002 | 10494 | 248.53 | 195.65 |
| | 1M003 | 10495 | 286.46 | 223.12 |
| | 1M004 | 10483 | 289.31 | 221.54 |
| | 1M006 | 10481 | 274.52 | 235.65 |
| | 1M007 | 10513 | 262.70 | 209.17 |
| | 1M008 | 10514 | 252.64 | 193.31 |
| | 1M009 | 10493 | 271.27 | 228.21 |
| S3 group | 2M002 | 10491 | 281.72 | 232.57 |
| | 2M003 | 10512 | 266.48 | 249.67 |
| | 2M004 | 10482 | 253.24 | 201.22 |
| | 2M005 | 10516 | 286.91 | 228.45 |
| | 2M007 | 10504 | 258.64 | 202.45 |
| | 2M008 | 10498 | 247.34 | 195.33 |
| | 2M009 | 10502 | 273.73 | 217.36 |
| t-PA group | 3M001 | 10501 | 289.15 | 230.57 |
| | 3M002 | 10499 | 246.68 | 203.58 |
| | 3M003 | 10488 | 263.91 | 203.63 |
| | 3M004 | 10503 | 259.23 | 199.35 |
| | 3M005 | 10477 | 273.31 | 205.31 |
| | 3M006 | 10489 | 281.77 | 223.28 |
| | 3M007 | 10497 | 281.59 | 223.30 |
| | 3M008 | 10496 | 273.94 | 217.60 |
| | 3M009 | 10507 | 251.02 | 200.68 |
| S3+t-PA group | 4M001 | 10505 | 288.39 | 247.50 |
| | 4M003 | 10508 | 272.53 | 219.13 |
| | 4M004 | 10511 | 273.92 | 218.05 |
| | 4M005 | 10509 | 283.71 | 218.91 |
| | 4M006 | 10478 | 263.60 | 202.12 |
| | 4M007 | 10484 | 266.20 | 210.77 |
| | 4M008 | 10479 | 248.95 | 199.20 |
| | 4M001 | 10505 | 288.39 | 247.50 |

The above descriptions are only preferred embodiments of the present disclosure, and are a limitation. Any modifications, equivalent replacements, improvements, etc. made within the spirit and principles of the present invention shall be included in the claimed protection scope.

**Claims**

1. A neuroprotective polypeptide compound, **characterized in that** it comprises a polypeptide having the following chemical formula and a salt thereof:
(M)m-(Lys-Leu-Ser-Ser-Ile-Glu/Asp-Ser/Thr-Asp/Glu-Val/Leu)-(N)n;

wherein m and n are integers from 0 to 3 but m and n are not simultaneously zero;
M is beta-Ala-His, beta-Ala-1-Methyl-His or beta-Ala-3-Methyl-His, and N is beta-Ala-His, beta-Ala-1-Methyl-His or beta-Ala-3-Methyl-His.

2. The neuroprotective polypeptide compound according to claim 1, **characterized in that** it comprises a polypeptide having the following chemical formula and a salt thereof:

beta-Ala-His-Lys-Leu-Ser-Ser-Ile-Glu/Asp-Ser/Thr-Asp/Glu-Val/Leu;
Lys-Leu-Ser-Ser-Ile-Glu/Asp-Ser/Thr-Asp/Glu-Val/Leu-beta-Ala-His;
beta-Ala-His-beta-Ala-His-Lys-Leu-Ser-Ser-Ile-Glu/Asp-Ser/Thr-Asp-Val/Leu;

Lys-Leu-Ser-Ser-Ile-Glu/Asp-Ser/Thr-Asp/Glu-Val/Leu-beta-Ala-His-beta-Ala-His;

beta-Ala-His-Lys-Leu-Ser-Ser-Ile-Glu/Asp-Ser/Thr-Asp/Glu-Val/Leu-beta-Ala-His;

beta-Ala-His-beta-Ala-His-beta-Ala-His-Lys-Leu-Ser-Ser-Ile-Glu/Asp-Ser/Thr-Asp-Val/Leu;

beta-Ala-His-beta-Ala-His-Lys-Leu-Ser-Ser-Ile-Glu/Asp-Ser/Thr-Asp-Val/Leu-beta-Ala-His;

beta-Ala-His-Lys-Leu-Ser-Ser-Ile-Glu/Asp-Ser/Thr-Asp/Glu-Val/Leu-beta-Ala-His-beta-Ala-His;

Lys-Leu-Ser-Ser-Ile-Glu/Asp-Ser/Thr-Asp/Glu-Val/Leu-beta-Ala-His-beta-Ala-His-beta-Ala-His;

wherein His is His, 1-Methyl-His or 3-Methyl-His.

3. The neuroprotective polypeptide compound according to claim 1, **characterized in that** it comprises a polypeptide having the following chemical formula and a salt thereof:

beta-Ala-His-Lys-Leu-Ser-Ser-Ile-Glu-Ser-Asp-Val;
beta-Ala-His-Lys-Leu-Ser-Ser-Ile-Asp-Ser-Asp-Val;
beta-Ala-His-Lys-Leu-Ser-Ser-Ile-Glu-Thr-Asp-Val;
beta-Ala-His-Lys-Leu-Ser-Ser-Ile-Glu-Thr-Glu-Val;
beta-Ala-His-Lys-Leu-Ser-Ser-Ile-Glu-Ser-Glu-Val;
beta-Ala-His-Lys-Leu-Ser-Ser-Ile-Asp-Thr-Asp-Val;
beta-Ala-His-Lys-Leu-Ser-Ser-Ile-Asp-Ser-Glu-Val;
beta-Ala-His-Lys-Leu-Ser-Ser-Ile-Asp-Thr-Glu-Val;
Lys-Leu-Ser-Ser-Ile-Glu-Ser-Asp-Val-beta-Ala-His;
Lys-Leu-Ser-Ser-Ile-Asp-Ser-Asp-Val-beta-Ala-His;
Lys-Leu-Ser-Ser-Ile-Glu-Thr-Asp-Val-beta-Ala-His;
Lys-Leu-Ser-Ser-Ile-Glu-Thr-Glu-Val-beta-Ala-His;
Lys-Leu-Ser-Ser-Ile-Glu-Ser-Glu-Val-beta-Ala-His;
Lys-Leu-Ser-Ser-Ile-Asp-Thr-Asp-Val-beta-Ala-His;
Lys-Leu-Ser-Ser-Ile-Asp-Ser-Glu-Val-beta-Ala-His;

Lys-Leu-Ser-Ser-Ile-Asp-Thr-Glu-Val-beta-Ala-His;
beta-Ala-(1-methyl-His)-Lys-Leu-Ser-Ser-Ile-Glu-Ser-Asp-Val;
beta-Ala-(1-methyl-His)-Lys-Leu-Ser-Ser-Ile-Asp-Ser-Asp-Val;
beta-Ala-(1-Methyl-His)-Lys-Leu-Ser-Ser-Ile-Glu-Thr-Asp-Val;
beta-Ala-(1-Methyl-His)-Lys-Leu-Ser-Ser-Ile-Glu-Thr-Glu-Val;
beta-Ala-(1-Methyl-His)-Lys-Leu-Ser-Ser-Ile-Glu-Ser-Glu-Val;
beta-Ala-(1-Methyl-His)-Lys-Leu-Ser-Ser-Ile-Asp-Thr-Asp-Val;
beta-Ala-(1-Methyl-His)-Lys-Leu-Ser-Ser-Ile-Asp-Ser-Glu-Val;
beta-Ala-(1-Methyl-His)-Lys-Leu-Ser-Ser-Ile-Asp-Thr-Glu-Val;
Lys-Leu-Ser-Ser-Ile-Glu-Ser-Asp-Val-beta-Ala-(1-Methyl-His);
Lys-Leu-Ser-Ser-Ile-Asp-Ser-Asp-Val-beta-Ala-(1-Methyl-His);
Lys-Leu-Ser-Ser-Ile-Glu-Thr-Asp-Val-beta-Ala-(1-Methyl-His);
Lys-Leu-Ser-Ser-Ile-Glu-Thr-Glu-Val-beta-Ala-(1-Methyl-His);
Lys-Leu-Ser-Ser-Ile-Glu-Ser-Glu-Val-beta-Ala-(1-Methyl-His);
Lys-Leu-Ser-Ser-Ile-Asp-Thr-Asp-Val-beta-Ala-(1-Methyl-His);
Lys- Leu-Ser-Ser- Ile-Asp-Ser-Glu- Val-beta-Ala-(1- Methyl-His);
Lys-Leu-Ser-Ser-Ile-Asp-Thr-Glu-Val-beta-Ala-(1-Methyl-His);
beta-Ala-(3-methyl-His)-Lys-Leu-Ser-Ser-Ile-Glu-Ser-Asp-Val;
beta-Ala-(3-methyl-His)-Lys-Leu-Ser-Ser-Ile-Asp-Ser-Asp-Val;
beta-Ala-(3-Methyl-His)-Lys-Leu-Ser-Ser-Ile-Glu-Thr-Asp-Val;
beta-Ala-(3-Methyl-His)-Lys-Leu-Ser-Ser-Ile-Glu-Thr-Glu-Val;
beta-Ala-(3-Methyl-His)-Lys-Leu-Ser-Ser-Ile-Glu-Ser-Glu-Val;
beta-Ala-(3-Methyl-His)-Lys-Leu-Ser-Ser-Ile-Asp-Thr-Asp-Val;
beta-Ala-(3-Methyl-His)-Lys-Leu-Ser-Ser-Ile-Asp-Ser-Glu-Val;
beta-Ala-(3-Methyl-His)-Lys-Leu-Ser-Ser-Ile-Asp-Thr-Glu-Val;
Lys-Leu-Ser-Ser-Ile-Glu-Ser-Asp-Val-beta-Ala-(3-Methyl-His);
Lys-Leu-Ser-Ser-Ile-Asp-Ser-Asp-Val-beta-Ala-(3-Methyl-His);
Lys-Leu-Ser-Ser-Ile-Glu-Thr-Asp-Val-beta-Ala-(3-Methyl-His);
Lys-Leu-Ser-Ser-Ile-Glu-Thr-Glu-Val-beta-Ala-(3-Methyl-His);
Lys-Leu-Ser-Ser-Ile-Glu-Ser-Glu-Val-beta-Ala-(3-Methyl-His);
Lys-Leu-Ser-Ser-Ile-Asp-Thr-Asp-Val-beta-Ala-(3-Methyl-His);
Lys-Leu-Ser-Ser-Ile-Asp-Ser-Glu-Val-beta-Ala-(3-Methyl-His);
Lys-Leu-Ser-Ser-Ile-Asp-Thr-Glu-Val-beta-Ala-(3-Methyl-His).

4. The neuroprotective polypeptide compound according to claim 1, **characterized in that** it comprises a polypeptide having the following chemical formula and a salt thereof:

beta-Ala-His-Lys-Leu-Ser-Ser-Ile-Glu-Ser-Asp-Val;
Lys-Leu-Ser-Ser-Ile-Glu-Ser-Asp-Val-beta-Ala-His;
beta-Ala-(1-methyl-His)-Lys-Leu-Ser-Ser-Ile-Glu-Ser-Asp-Val;
Lys-Leu-Ser-Ser-Ile-Glu-Ser-Asp-Val-beta-Ala-(1-Methyl-His);
beta-Ala-(3-methyl-His)-Lys-Leu-Ser-Ser-Ile-Glu-Ser-Asp-Val;
Lys-Leu-Ser-Ser-Ile-Glu-Ser-Asp-Val-beta-Ala-(3-Methyl-His).

5. The neuroprotective polypeptide compound according to claim 1, **characterized in that** it comprises a polypeptide having the following chemical formula and a salt thereof:

beta-Ala-His-Lys-Leu-Ser-Ser-Ile-Glu-Ser-Asp-Val;
Lys-Leu-Ser-Ser-Ile-Glu-Ser-Asp-Val-beta-Ala-His.

6. The neuroprotective polypeptide compound according to claim 1, **characterized in that** it comprises a polypeptide having the following chemical formula and a salt thereof:
beta-Ala-His-Lys-Leu-Ser-Ser-Ile-Glu-Ser-Asp-Val.

7. The neuroprotective polypeptide compound according to any one of claims 1 to 6 for use in medicine.

8. The neuroprotective polypeptide compound according to any one of claims 1 to 6 for use in treating neurological diseases, such as ischemic stroke, hemorrhagic stroke, brain trauma, Alzheimer's disease, Parkinson's disease and other neurodegenerative diseases.

9. A medicament comprising the neuroprotective polypeptide compound according to any one of claims 1 to 6, such as for injection, oral, sublingual, spray or anal administration.

10. A combination of the neuroprotective polypeptide compound according to any one of claims 1 to 6 and a thrombolytic drug.

11. The combination of any one of claim 10 for use in the treatment of neurological diseases, such as ischemic stroke, hemorrhagic stroke, brain trauma, Alzheimer's disease, Parkinson's disease and other neurodegenerative diseases.

⟩ MCAO model group

Fig. 1

⟩ S1

Fig. 2

⟩ S3

Fig. 3

Fig. 4

Fig. 5

Fig. 6

Fig. 7

Fig. 8

Fig. 9

Fig. 10

## INTERNATIONAL SEARCH REPORT

| International application No. |
|---|
| **PCT/CN2022/109048** |

**A. CLASSIFICATION OF SUBJECT MATTER**

C07K 7/06(2006.01)i; C07K 7/08(2006.01)i; A61K 38/08(2019.01)i; A61K 38/10(2006.01)i; A61P 25/00(2006.01)i; A61P 9/12(2006.01)i; A61P 25/28(2006.01)i; A61P 25/16(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNKI, CNABS, CNTXT, WPABS, WPABSC, DWPI, ENTXT, ENTXTC, ISI web of science: 神经保护, 肽, 突触后密度蛋白, neuroprotection, peptides , PSD-95, NR2B9c, 肌肽, L-Carnosine, 鹅肌肽, Anserine, 鲸肌肽, 蛇肌肽, Balenine, Ophidine等; GENBANK, EMBL, STN, 中国专利生物序列检索系统和检索的序列, Chinese Patent Biological Sequence Retrieval System and searched sequences: 权利要求中的序列, sequences in the claims.

**C. DOCUMENTS CONSIDERED TO BE RELEVANT**

| Category* | Citation of document, with indication, where appropriate, of the relevant passages | Relevant to claim No. |
|---|---|---|
| Y | CN 106267149 A (NANJING MEDICAL UNIVERSITY) 04 January 2017 (2017-01-04) claims 1-4, and description, paragraph [0003] | 1-11 |
| Y | CN 113150065 A (WUHAN INNERSE PHARMACEUTICAL INC.) 23 July 2021 (2021-07-23) claims 1-10, and description, paragraphs [0002]-[0003] | 1-11 |
| Y | CN 103648518 A (NONO INC.) 19 March 2014 (2014-03-19) abstract, and claims 1-15 | 10-11 |
| Y | CN 106265594 A (NANJING MEDICAL UNIVERSITY) 04 January 2017 (2017-01-04) claims 1-2, and description, paragraph [0003] | 1-11 |
| PX | CN 113735938 A (INNERSE (ZHUHAI) PHARMACEUTICAL CO., LTD.) 03 December 2021 (2021-12-03) claims 1-9 | 1-9 |
| PY | CN 113735938 A (INNERSE (ZHUHAI) PHARMACEUTICAL CO., LTD.) 03 December 2021 (2021-12-03) claims 1-9 | 10-11 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 October 2022** | **28 October 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/109048**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] With regard to any nucleotide and/or amino acid sequence disclosed in the international application, the present report was completed to the extent that a meaningful search can be conducted in the case that no sequence table complies with WIPO Standard ST.26.

Form PCT/ISA/210 (continuation of first sheet) (January 2015)

## INTERNATIONAL SEARCH REPORT

| International application No. |
|---|
| **PCT/CN2022/109048** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **7-8**
   because they relate to subject matter not required to be searched by this Authority, namely:

   [1]   PCT Rule 39.1(iv)-methods for the treatment of a human or animal body by surgery or therapy, as well as diagnostic methods.

   [2]   The search for claims 7-8 was conducted on the basis of the reasonably expected subject matter, i.e., an application of the neuroprotective polypeptide compound according to any one of claims 1-6 in the preparation of a drug.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/109048**

| Patent document cited in search report | | | Publication date (day/month/year) | Patent family member(s) | | | Publication date (day/month/year) |
|---|---|---|---|---|---|---|---|
| CN | 106267149 | A | 04 January 2017 | None | | | |
| CN | 113150065 | A | 23 July 2021 | None | | | |
| CN | 103648518 | A | 19 March 2014 | DK | 2723363 | T3 | 15 October 2018 |
| | | | | RS | 57798 | B1 | 31 December 2018 |
| | | | | EP | 2723363 | A2 | 30 April 2014 |
| | | | | PL | 2723363 | T3 | 31 December 2018 |
| | | | | JP | 2017193535 | A | 26 October 2017 |
| | | | | US | 2014248257 | A1 | 04 September 2014 |
| | | | | KR | 20210109661 | A | 06 September 2021 |
| | | | | HU | E040564 | T2 | 28 March 2019 |
| | | | | KR | 20140053971 | A | 08 May 2014 |
| | | | | SI | 2723363 | T1 | 31 December 2018 |
| | | | | EP | 3427748 | A1 | 16 January 2019 |
| | | | | AU | 2012274910 | A1 | 16 January 2014 |
| | | | | US | 2019091283 | A1 | 28 March 2019 |
| | | | | KR | 20190107195 | A | 18 September 2019 |
| | | | | PT | 2723363 | T | 08 November 2018 |
| | | | | CY | 1121379 | T1 | 29 May 2020 |
| | | | | WO | 2012176172 | A2 | 27 December 2012 |
| | | | | ES | 2912042 | T3 | 24 May 2022 |
| | | | | HU | E058238 | T2 | 28 July 2022 |
| | | | | LT | 2723363 | T | 25 October 2018 |
| | | | | KR | 20220106862 | A | 29 July 2022 |
| | | | | EP | 4052721 | A1 | 07 September 2022 |
| | | | | HR | P20181526 | T1 | 14 December 2018 |
| | | | | US | 2021308209 | A1 | 07 October 2021 |
| | | | | PT | 3427748 | T | 03 May 2022 |
| | | | | PL | 3427748 | T3 | 30 May 2022 |
| | | | | HR | P20220519 | T1 | 27 May 2022 |
| | | | | JP | 2020189859 | A | 26 November 2020 |
| | | | | DK | 3427748 | T3 | 19 April 2022 |
| | | | | ES | 2690994 | T3 | 23 November 2018 |
| | | | | CA | 2839630 | A1 | 27 December 2012 |
| | | | | KR | 20200092437 | A | 03 August 2020 |
| | | | | JP | 2019038831 | A | 14 March 2019 |
| | | | | JP | 2014520142 | A | 21 August 2014 |
| CN | 106265594 | A | 04 January 2017 | None | | | |
| CN | 113735938 | A | 03 December 2021 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8536129 B, A. Tasker, T. Doucette, M. Tymianski, K. Mendoza, M. P. Belmares, D. Garman, and P. S. Lu

- US 8940699 B2, Michael Tymianski and Jonathan D. Garman
- GB 149252010 A

### Non-patent literature cited in the description

- **MICHAEL D HILL et al.** Efficacy and safety of nerinetide for the treatment of acute ischaemic stroke (ESCAPE-NA1): a multicentre, double-blind, randomized controlled trial. *Lancet,* 2020, vol. 395 (10227), 878-887

- **MICHAEL D HILL et al.** Efficacy and safety of nerinetide for the treatment of acute ischaemic stroke (ESCAPE-NA1): a multicentre, double-blind, randomised controlled trial. *Lancet,* 20 February 2020